# EUROPEAN PATENT SPECIFICATION

(11) **EP 3 117 901 B1**
(45) Date of publication and mention of the grant of the patent: **01.05.2024**
(21) Application number: 15761960.2
(22) Date of filing: 10.03.2015
(51) Int. Cl.: B01J 23/80, B01J 35/10, B01J 37/03, C07B 61/00, C07C 209/36, C07C 211/46

(54) **HYDROGENATION CATALYST FOR AROMATIC NITRO COMPOUND, AND METHOD FOR MANUFACTURING SAME**
HYDRIERUNGSKATALYSATOREN FÜR AROMATISCHE NITROVERBINDUNG UND VERFAHREN ZUR HERSTELLUNG DAVON
CATALYSEUR D'HYDROGÉNATION POUR COMPOSÉ NITRÉ AROMATIQUE, ET PROCÉDÉ DE FABRICATION DE CELUI-CI

(30) Priority: 11.03.2014 JP 2014048046
(43) Date of publication of application: 18.01.2017
(73) Proprietor: Tosoh Corporation, Shunan-shi, Yamaguchi 746-8501 (JP)
(72) Inventor: KAWABE, Shou, Shunan-shi Yamaguchi 746-8501 (JP); HARADA, Tsuneo, Shunan-shi Yamaguchi 746-8501 (JP); FUJIWARA, Tomiyoshi, Shunan-shi Yamaguchi 746-8501 (JP)
(74) Representative: Vigand, Philippe
(86) International application number: PCT/JP2015/056924
(87) International publication number: WO 2015/137301

(56) References cited:
- EP-A1- 0 646 410
- EP-A1- 2 517 789
- EP-A1- 2 784 056
- EP-A2- 1 338 587
- WO-A1-2008/044707
- WO-A1-2013/015889
- FR-A1- 2 525 591
- FR-A1- 2 525 591
- JP-A- H05 337 371
- JP-A- H07 100 381
- JP-A- 2007 289 855
- US-A1- 2012 178 975
- US-A1- 2013 199 968

## Description

### TECHNICAL FIELD

The present invention relates to a hydrogenation catalyst for an aromatic nitro compound. In particular, the present invention relates to a composition for a catalyst to be used in a method for hydrogenating nitrobenzene to produce aniline.

### BACKGROUND ART

As a catalyst to be used for a reaction to hydrogenate nitrobenzene by a gas phase reaction to produce aniline, for example, a catalyst containing chromium oxide has been reported (Patent Document 1).

Patent Document 1 discloses a process for preparing aniline by subjecting gaseous nitrobenzene to hydrogen reduction by using a copper/chromium oxide catalyst at a reaction temperature of from 180 to 370°C under a pressure of from 0.1 to 0.5 MPaG and under condition of a nitrobenzene concentration of from 2 to 14 vol%. However, the catalyst containing chromium may cause health hazards and environmental pollution. Therefore, in addition to careful attention required in handling, significant labor and expense were required for the treatment and recovery of used catalyst.

Whereas, as catalysts easy to handle, catalysts containing no chromium oxide have been reported (Patent Documents 2 to 4). Patent Document 2 proposes a molded hydrogenation catalyst comprising copper, calcium silicate and natural clay minerals, and a method for hydrogenating an aromatic nitro compound by using it. Further, Patent Document 3 proposes a molded catalyst composed of a hydrotalcite comprising copper, calcium silicate and magnesium, and a method for hydrogenating an aromatic nitro compound by using it.

Further, Patent Document 4 proposes a catalyst comprising a ceramic support material having a BET specific surface area of less than 40 m²/g, and, as incorporated therein, at least one metal in Groups Vllla, lb, Ilb, IVa, Va, VIa, IVb and Vb in the Periodic Table of elements, and a method for producing an aromatic amine by using it.

### PRIOR ART DOCUMENTS

### PATENT DOCUMENTS

Patent Document 1: JP-A-49-000231
Patent Document 2: JP-A-11-507867
Patent Document 3: JP-A-2007-289855
Patent Document 4: JP-A-2001-505893

WO 2013/048279 A discloses a dehydrogenation catalyst useful in a method of catalytic N-alkylation of aromatic amines and nitro-compound, said catalyst comprising 55 wt% CuO, 10.5 wt% ZnO, 13.5 wt% Cr₂O₃, the balance being Al₂O₃.

EP 2 517 789 A discloses possible alternative support materials for copper containing hydrogenation catalysts, among which calcium silicate is described as the most preferred.

### DISCLOSURE OF INVENTION

### TECHNICAL PROBLEM

The catalysts disclosed in Patent Documents 2 to 4 are improved over the prior art in selectivity and yield of the aromatic amine compounds, but not improved in catalyst lifetime. Therefore, in order to carry out the reaction at a high conversion with these catalysts, it was necessary to change the catalysts frequently.

It is an object of the present invention to provide a composition which, in a catalytic reaction, especially in a hydrogenation reaction of an aromatic nitro compound, exhibits a high raw material conversion equal to the prior art and enables a consistent catalytic reaction for a long period of time. Further, it is another object of the present invention to provide a catalyst comprising such a composition, as well as a method for producing an aromatic amine compound and a method for hydrogenating an aromatic nitro compound, by using such a composition.

### SOLUTION TO PROBLEM

The present inventors have conducted intensive studies in order to solve the above problem. As a result, they have found that a composition comprising copper, zinc and at least one member selected from the group consisting of aluminum oxide, silicon oxide, calcium oxide and their composite oxides, can suppress coking in the catalytic reaction, and thus have arrived at accomplishing the present invention.

That is, the various aspects of the present invention are as follows.

In a first aspect, the present invention relates to a hydrogenation catalyst consisting essentially of from 20 to 50 wt% of copper, from 1 to 15 wt% of zinc, optionally an auxiliary catalyst component selected from the group consisting of magnesium, strontium, barium and aluminum, and calcium silicate wherein the molar ration of calcium oxide to silicon oxide in the calcium silicate is from 0.1 to 0.7, said hydrogenation catalyst having a BET specific surface area of from 3 to 30 m²/g.

The hydrogenation catalyst according to said first aspect preferably contains at least one member selected from the group consisting of magnesium, strontium, barium and aluminum.

In a second aspect, the present invention relates to a method for producing the hydrogenation catalyst as defined in the above first aspect, characterized by comprising a mixing step of contacting calcium silicate with an aqueous solution containing copper and zinc, to obtain a mixture, a molding step of molding the mixture, and a calcination step of calcining the mixture at a temperature of at least 680°C and less than 800°C for 1 hour to 24 hours.

In a third aspect, the present invention relates to a method for producing an aromatic amine compound, which comprises contacting the hydrogenation catalyst as defined in the above first aspect with raw material comprising an aromatic nitro compound and hydrogen.

### ADVANTAGEOUS EFFECTS OF INVENTION

The present invention can provide a composition which, in a catalytic reaction, especially in a hydrogenation reaction of an aromatic nitro compound, exhibits a high raw material conversion equal to the prior art and enables a consistent catalytic reaction for a long period of time. Further, the present invention can provide a catalyst comprising such a composition, and a method for producing an aromatic amine compound by using the composition of the present invention.

If the composition of the present invention is used as a catalyst, especially in a hydrogenation reaction of an aromatic nitro compound, for example, in a method for producing aniline by hydrogenating nitrobenzene, coking is less likely to occur, and an increase in pressure loss of the catalyst layer is suppressed. As a result, it becomes possible to conduct a consistent catalytic reaction for a long period of time.

### DESCRIPTION OF EMBODIMENTS

The composition of the present invention contains copper. Copper is considered to be a main component for expressing the hydrogenation reaction activity. The higher the content of copper, the higher the hydrogenation reaction activity. However, if the content of copper is too high, excessive hydrogenation by-products tend to be formed. Further, if the copper concentration is too high, when the composition of the present invention is made into a molded product, its strength tends to be low. If the content of copper is too small, another side reaction is likely to occur, due to e.g. silicon oxide to be used as a carrier. Therefore, the content of copper is from 20 to 50 wt%, preferably from 20 to 45 wt%, more preferably from 23 to 41 wt%, as the amount calculated as copper metal per the total weight of the composition. Since coking is thereby particularly inhibited, the content of copper is particularly preferably from 31 to 41 wt%.

The composition of the present invention contains zinc. Zinc has effects such as an activity-promoting effect, an effect to prevent sintering of copper, etc. In addition to these, zinc in the composition of the present invention has an effect to prevent coking for a long period of time. The composition of the present invention thereby tends to have high coking resistance.

Here, coking is such a phenomenon that a highly slaked carbon-rich by-product (coke) is formed and deposited on the catalyst. If the catalyst is covered with coke, the catalytic activity is reduced. In particular, in a hydrogenation reaction of an aromatic nitro compound by a vapor phase reaction using a fixed bed catalytic reactor, if coking progresses, in addition to a decrease in the activity of the catalyst, the differential pressure of the catalyst layer (hereinafter referred to also as the "pressure loss") will increase. As a result, it becomes difficult to continue the reaction consistently. The composition of the present invention contains zinc, whereby such coking can be significantly suppressed. Therefore, when the composition of the present invention is used as a catalyst, it is not required to shutdown the operation during the reaction or to change the catalyst accompanying it. Thus, much labor and costs are not required, and the productivity of the catalytic reaction product will be significantly improved.

If zinc is too much, a side reaction tends to increase. If the content of zinc is too high, when the composition of the present invention is formed into a molded product, its strength tends to be low. Therefore, the content of zinc is from 1 to 15 wt%, preferably from 4 to 10 wt%, more preferably from 4.0 to 9 wt%, as the amount calculated as zinc metal per the total weight of the composition. Since coking resistance becomes particularly high, the content of zinc is particularly preferably from 4.0 to 8.7 wt%.

Within the above ranges, the content of copper and the content of zinc may be a combination of any ranges.

The composition of the present invention preferably contains a component (hereinafter referred to also as an "auxiliary catalyst component") to further improve the raw material conversion or the reaction rate. As such a component, at least one member selected from the group consisting of magnesium, strontium, barium and aluminum, may be mentioned, and either magnesium or aluminum is preferred.

The content of the auxiliary catalyst component may be in a range where the side reaction is suppressed, and when the composition is made into a molded product, the strength does not become insufficient. The auxiliary catalyst component is preferably contained in an amount of from 0.1 to 10 wt%, more preferably from 0.1 to 5 wt%, further preferably from 0.2 to 3.5 wt%, as the amount calculated as metal per the total weight of the composition.

When the auxiliary catalyst component is magnesium, strontium or barium, the content may be preferably from 0.1 to 3.0 wt%, more preferably from 0.1 to 1.0 wt%. When the auxiliary catalyst component is aluminum, the preferred content may be from 1 to 5 wt%.

The composition of the present disclosure contains an oxide (hereinafter referred to also as a "carrier oxide") containing at least one member selected from the group consisting of aluminum, silicon and calcium aluminum. The carrier oxide, i.e. aluminum oxide, silicon oxide, calcium oxide, as well as a composite oxide containing at least two elements selected from aluminum, silicon and calcium, will function as a socalled catalyst carrier.

The content of the carrier oxide to be contained in the composition of the present disclosure or invention may be the balance of zinc and copper. Further, when an auxiliary catalyst component is contained, the carrier oxide may be the balance of zinc, copper and the auxiliary catalyst component. For example, the weight ratio of the carrier oxide to the weight of the composition of the present disclosure or invention may be from 21 to 65 wt%.

The carrier oxide may be a mixture of at least two members selected from the group consisting of aluminum oxide, calcium oxide and silicon oxide, i.e. a physical mixture of at least two members selected from the group consisting of aluminum oxide, calcium oxide and silicon oxide. However, the carrier oxide is preferably a composite oxide containing any two or more elements selected from aluminum, silicon and calcium, since copper and zinc thereby tend to be more uniform in the composition of the present disclosure. As a composite oxide as a specific carrier oxide, at least one member selected from the group consisting of a composite oxide of aluminum and silicon, a composite oxide of aluminum and calcium, a composite oxide of calcium and silicon, and a composite oxide of calcium, silicon and aluminum, may be exemplified. In accordance with the present invention, the carrier oxide is a composite oxide of calcium and silicon, calcium silicate, since the catalyst lifetime thereby becomes longer, when the composition of the present invention is made into a catalyst. Further, in accordance with the present invention, the composition of the calcium silicate is such that when silicon and calcium in the calcium silicate are calculated as oxides, the molar ratio of CaO to SiO₂ in the calcium silicate (hereinafter referred to also as "CaO/SiO₂") is at least 0.1 and at most 0.7.

In a case where the carrier oxide contains calcium oxide, the amount calculated as calcium (Ca) per the total weight of the composition is preferably from 5 to 10 wt%, more preferably from 5 to 9 wt%.

The composition of the present invention consists essentially of the above components. As it is made of a composition comprising copper, zinc and calcium silicate as the carrier oxide, and, as the case requires, the auxiliary catalyst component, the composition of the present invention is likely to become a catalyst having a longer catalyst lifetime.

On the other hand, natural mineral components such as attapulgite, kaolin, etc. tend to have their composition changeable. In order to obtain a catalyst having characteristics stabilized, it is preferred that the composition of the invention contains no natural mineral component. Further, heretofore, a hydrogenation catalyst containing magnesium oxide derived from hydrotalcite has been known. However, the composition of the present invention has high coking resistance, even if it does not contain such magnesium oxide. Further, magnesium oxide derived from hydrotalcite tends to aggregate and thus is poor in dispersibility, whereby there is a risk that coking resistance of the composition of the present invention will be impaired. Therefore, the composition of the present invention preferably contains no magnesium oxide derived from hydrotalcite.

The BET specific surface area of the composition of the present invention is from 3 to 30 m²/g, preferably from 5.5 to 25 m²/g, more preferably from 7 to 25 m²/g. With the BET specific surface area of at least 3 m²/g, the composition of the present invention has a practical catalytic activity. Conversely, with the BET specific surface area of at most 30 m²/g, the composition of the present invention has a practical high catalytic activity, and yet it can inhibit coking. In order to obtain a higher catalytic activity and coking resistance, the BET specific surface area is preferably from 7 to 24 m²/g.

The compositions of the present invention preferably has a density of at least 1.5 g/mL and at most 2.5 g/mL. When the density of the molded product is at least 1.5 g/mL, the composition of the present invention tends to be a catalyst having a high raw material conversion. On the other hand, when the density of the molded product is at most 2.5 g/mL, it tends to be a catalyst whereby the hydrogenation reaction proceeds without causing side reactions. In order to let the hydrogenation reaction proceed highly efficiently, the density of the catalyst of the present invention is preferably from 1.7 g/mL to 2.5 g/mL, more preferably from 1.8 g/mL to 2.5 g/mL, further preferably from 1.8 g/mL to 2.3 g/mL.

The shape of the composition of the present invention is optional, and it may be any shape and size so long as it can be used as a catalyst. For example, when using the composition of the present invention as a hydrogenation catalyst, it may be in the form of a powder or a molded product.

The shape of the compositions of the present invention is preferably a molded product, since the pressure loss is thereby small. The shape of the molded product may, for example, be spherical, substantially spherical, cylindrical, disk-shaped, cone-shaped, polyhedral or indefinite shape. The size of the molded product may be any size depending upon the scale of the reaction, and may, for example, be preferably from 1 to 10 mm, more preferably from 1 to 7 mm, further preferably from 1 to 6 mm.

The composition of the present invention has high coking resistance. Therefore, the coking rate of the composition of the present invention is preferably at most 0.2 g/hr/L, more preferably at most 0.1 g/hr/L, further preferably at most 0.05 g/hr/L, still further preferably at most 0.03 g/hr/L. It is desirable that no coking takes place, i.e. the coking rate is 0 g/hr/L, but, usually, it is at least 0.0005 g/hr/L, typically at least 0.001 g/hr/L.

Here, the coking rate is an index for coking resistance and is a value obtained from the following formula. Coking rate (g/hr/L) = Weight increase of the composition (g)/reaction time (hr)/catalyst volume (L)

In the above formula, the catalyst volume is the volume of the catalyst layer made from the catalyst comprising the composition of the present invention and used in the catalytic reaction. The weight increase of the composition is a value obtained from the following formula. Weight increase of the composition (g) = weight of the composition in the catalyst layer after reaction (g) - wight of the composition in the catalyst layer before the reaction (g)

The composition of the present disclosure may be obtained by a production method comprising a mixing step of contacting an oxide (carrier oxide) containing at least one element selected from the group consisting of aluminum, silicon and calcium, with an aqueous solution containing copper and zinc, to obtain a mixture, and a calcination step of calcining the mixture.

In the mixing step, an oxide (carrier oxide) containing at least one element selected from the group consisting of aluminum, silicon and calcium, is contacted with an aqueous solution containing copper and zinc. A mixture of copper and zinc, and the carrier oxide, is thereby obtained.

The aqueous solution containing copper and zinc, to be subjected to the mixing step (hereinafter referred to also as the "supported metal aqueous solution") is obtainable by dissolving water-soluble salts of copper and zinc in water. Further, in the case of obtaining a composition containing an auxiliary catalyst component, the supported metal aqueous solution may contain at least one water-soluble salt selected from the group consisting of magnesium, strontium, barium and aluminum, and further preferably contains at least either water-soluble salt of magnesium or aluminum.

As the water-soluble salt of copper, zinc or an auxiliary catalyst component, at least one member selected from the group consisting of a chloride, a bromide, a nitrate, an acetate and a sulfate of each of them, may be exemplified.

The concentrations of copper, zinc and the auxiliary catalyst component in the supported metal aqueous solution, may be suitably adjusted depending on the desired contents of copper, zinc and the auxiliary catalyst component in the composition. For example, the copper concentration in the supported metal aqueous solution may be from 5 to 15 wt%, further from 8 to 12 wt%, and the zinc concentration may be from 0.1 to 5 wt%, further from 0.1 to 2.5 wt%. The concentration of the auxiliary catalyst component in the supported metal aqueous solution may, for example, be from 0.1 to 3 wt%. More specifically, the aluminum concentration may be from 0.1 to 1.5 wt%, and the magnesium concentration may be from 0.1 to 1 wt%.

The carrier oxide may be an oxide containing at least one element selected from the group consisting of aluminum, silicon and calcium. As the carrier oxide, one obtained by any method may be used. For example, when using calcium silicate in accordance with the present invention as the carrier oxide, it is preferably calcium silicate obtained by mixing a silica source and a calcium source.

Such calcium silicate is obtainable by suspending a silica source in water, and mixing a calcium source thereto to prepare a calcium silicate slurry, or by suspending a silica source in water, and adding a calcium source thereto. The mixing of the calcium source and the silica source is preferably carried out at a temperature of from 20 to 60°C, more preferably from 30 to 50°C, since the reaction efficiency thereby becomes high.

The silica source is preferably at least one member selected from the group consisting of precipitated silica, silica gel, amorphous silica and fumed silica, more preferably at least either silica gel or amorphous silica.

The calcium source may be at least one member selected from the group consisting of calcium carbonate, calcium chloride, quicklime (calcium oxide: CaO) and slaked lime (calcium hydroxide: Ca (OH)₂). The calcium source is preferably slaked lime, since the reactivity is high.

The method for mixing the supported metal aqueous solution and the carrier oxide may be any method so long as mixing can be uniformly done. For example, any method such as dry mixing, wet mixing, co-precipitation, impregnation, or ion exchange method, may be employed.

As a preferred mixing method, a method may be mentioned wherein the slurry containing the carrier oxide, and the supported metal aqueous solution, are mixed to obtain a suspension, which is then cleaned and dried.

The preferred mixing method will be described specifically with reference to a case where the carrier oxide is calcium silicate in accordance with the present invention. The supported metal aqueous solution is added into a calcium silicate slurry. At that time, the pH of the calcium silicate slurry is adjusted to be from 6 to 8. Thereby, the hydroxide or carbonate of the metal to be supported will be precipitated on calcium silicate. For the pH adjustment of the calcium silicate slurry, an alkaline aqueous solution of sodium carbonate, potassium carbonate, sodium hydroxide or potassium hydroxide, may be used.

The temperature at the time of precipitating the hydroxide or carbonate of the metal to be supported, is not particularly limited and may be suitably set at a level not to impair the operability and safety of the operation. The temperature for the mixing may, for example, be from 10 to 50°C.

Further, the addition speed of the supported metal aqueous solution is also not particularly limited. However, if the addition speed is too fast, particles of precipitates tend to be too fine, whereby it may take time more than necessary for solid-liquid separation. Therefore, the addition time of the supported metal aqueous solution may be set to be preferably from 4 to 8 hours, more preferably from 5 to 8 hours.

The obtained suspension is subjected to solid-liquid separation and washing with water to obtain a cake, followed by drying to obtain a mixture. For the drying, it is preferred to set the conditions so that the weight loss upon heating the mixture at 150°C would be at most 5%. This makes powdering less likely to occur when the mixture is calcined. Further, in a case where the mixture is formed into a molded product, breakage is less likely to occur when it is calcined. As such drying conditions, the treatment may be carried out at from 100 to 130°C for from 2 to 48 hours in the atmosphere.

In a case where the obtainable composition is to be formed into a molded product, it is preferred to have a molding step of molding the mixture after the mixing step.

The molding method may be any molding method such as extrusion molding, granulation molding, or tableting molding. From the viewpoint of the strength of a molded product, the molding method is preferably tableting molding.

In the molding step, a molding aid such as a lubricant may be used as the case requires. As the molding aid, at least one member selected from the group consisting of graphite, organic celluloses and lignins, may be mentioned. Here, the molding aid may be in an amount of from 1 to 5 wt% relative to the weight of the mixture.

The density of the molded product obtainable in the molding step is preferably from 0.9 to 1.5 g/mL. When the density of the molded product is at least 0.9 g/mL, more preferably at least 1.0 g/mL, further preferably at least 1.1 g/mL, the filling property of the composition after the calcining tends to be high. Further, when the density of the molded product is made to be at most 1.5 g/mL, more preferably at most 1.45 g/mL, the composition after the calcining will have a practical filling property.

The shape of the molded product is not particularly limited. The shape of the molded product may be such a shape as spherical, substantially spherical, cylindrical, disk-shaped, cone-shaped, polyhedral or indefinite shape.

In the calcination step, a mixture of copper and zinc, and the carrier oxide, is calcined. By calcining the mixture, it is possible to obtain a composition suitable as a catalyst, specifically as a hydrogenation catalyst, more specifically as a hydrogenation catalyst for an aromatic nitro compound. Because it is simple, the calcining is preferably conducted in air.

Further, the calcining is preferably conducted at such a temperature and time that the BET specific surface area of the composition would be within the above-mentioned range. As such a temperature, preferably at least 680°C and less than 800°C, more preferably at least 690°C and less than 800°C, further preferably at least 695°C and at most 775°C, may be mentioned. A preferred calcining time varies depending on the calcining temperature, but, the calcining time may be preferably from 1 hour to 24 hours, more preferably from 1 hour to 12 hours, further preferably from 1 hour to 6 hours.

The composition of the present invention can be used as a catalyst, specifically as a hydrogenation catalyst, more specifically as a hydrogenation catalyst for an aromatic nitro compound. The catalyst comprising the composition of the present invention (hereinafter referred to also as the "catalyst of the present invention") has a high coking resistance. It is thereby possible to prevent an increase in pressure loss, and to carry out a catalytic reaction consistently. In addition, the frequency to change the catalyst is reduced, and it is possible to improve the efficiency for production of e.g. an aromatic amine compound.

The catalyst of the present invention can produce an aromatic amine compound by contacting it with raw material comprising an aromatic nitro compound and hydrogen. That is, by the hydrogenation of an aromatic nitro compound, it is possible to produce an aromatic amine compound corresponding to the aromatic nitro compound. In such a case, the catalyst of the present invention may be a hydrogenation catalyst for an aromatic nitro compound, characterized by comprising a) from 20 to 50 wt% of copper, b) from 1 to 15 wt% of zinc, c) from 2 to 10 wt% of at least one alkaline earth metal selected from magnesium, calcium, strontium and barium, and d) the rest being aluminum oxide, silicon oxide and/or their composite oxide.

The aromatic nitro compound may be at least one member selected from the group consisting of nitrobenzene, alkyl-substituted nitrobenzenes, nitrophenols, nitropyridines and nitrochlorobenzenes, preferably nitrobenzene.

Further, the corresponding aromatic amine compound may, for example, be at least one member selected from the group consisting of aniline, alkyl-substituted anilines, aminophenols, aminopyridines and aminochlorobenzenes, preferably aniline.

The catalyst of the present invention can be used as either a fluidized bed catalyst or a fixed bed catalyst. Here, in the case of a fixed bed catalyst, the air space surrounding the catalyst is reduced by the progress of coking. Thus, the fixed-bed catalyst has problems such as an increase in the pressure loss, frequent suspensions of the reaction, and necessity for replacement of the catalyst associated therewith. Since these problems can be solved, the catalyst of the present invention may be used particularly preferably as a fixed bed catalyst.

The catalyst of the present invention is preferably used under gas phase reaction conditions.

As an example of a method for hydrogenating an aromatic nitro compound and a method for producing an aromatic amine compound, by using the catalyst of the present invention, a method for producing aniline by hydrogenating nitrobenzene by a gas phase reaction will be described.

It is possible to produce aniline by contacting the catalyst of the present invention with a gas containing nitrobenzene and hydrogen.

As the reactor, a known fixed-bed catalytic reactor or fluidized bed catalytic reactor may be used, but it is preferred to use a fixed-bed catalytic reactor. In the case of using a fixed bed catalytic reactor, the reaction heat is large, and therefore, it is preferred to use an apparatus provided with a sufficient heat removal mechanism.

For filling of the catalyst, only the catalyst of the present invention may be charged to the reactor. Otherwise, the catalyst of the present invention may be filled into the reactor as a catalyst in combination with another catalyst.

For example, the catalyst of the present invention is filled at the inlet portion of the raw material gas, and at the rest, a conventional catalyst may be filled.

As between the catalyst in the vicinity of the inlet portion of the raw material gas (hereinafter referred to also as the "upper catalyst") and the catalyst at other portions (hereinafter, also referred to as the "middle/lower catalyst"), the frequency of the hydrogenation reaction of nitrobenzene (hereinafter simply referred to also as the "reaction") is different. At the upper catalyst, the frequency of the reaction is high, and therefore, coking is likely to occur. On the other hand, at the middle/lower catalyst, the reaction proceeds mildly as compared to at the upper catalyst. By using the catalyst of the present invention as the catalyst in the vicinity of the inlet portion, it is possible to let the reaction proceed in high frequency without causing coking. Thus, the middle/lower catalyst being less susceptible to the influence of coking, tends to have a higher raw material conversion and higher selectivity, than when it is used alone, and further, the catalyst lifetime can be prolonged.

In such a case, the middle/lower catalyst is preferably a catalyst having the same level of the raw material conversion and selectivity as the catalyst of the present invention. Such a catalyst may, for example, be a catalyst composed of copper and calcium silicate wherein the molar ratio of calcium oxide to silicon oxide in the calcium silicate is from 0.1 to 0.7. In such a catalyst, the concentration of copper is preferably from 20 to 60 wt%, and the BET specific surface area is preferably at least 100 m²/g.

Further, the catalyst of the present invention and a conventional catalyst may be used as a mixture. Furthermore, the catalyst of the present invention and a catalyst which is higher in either the raw material conversion or the selectivity than the catalyst of the present invention (hereinafter referred to also as a "high activity catalyst") may be mixed for use.

By mixing the high activity catalyst and the catalyst of the present invention, the reaction load of the high activity catalyst can be suppressed. Thus, as compared with the case of using it alone, it is possible to prolong the catalyst lifetime of the high activity catalyst itself.

It is possible to produce aniline by hydrogenating nitrobenzene by using the catalyst of the present invention. In the case of producing aniline by using the catalyst of the present invention, before carrying out the reaction, the catalyst of the present invention is reduced by contact with hydrogen gas at a temperature of from 150 to 300°C, whereby the catalyst is activated. Here, instead of hydrogen gas, one obtained by diluting hydrogen gas with an inert gas may be used.

In the hydrogenation reaction of nitrobenzene, a mixture gas of pre-heated nitrobenzene and hydrogen may be used as a raw material gas, to be fed to the reactor. Otherwise, a gas having an inert gas such as nitrogen mixed to the above mixture gas may be used as the raw material gas. As the reaction conditions, the following conditions may be mentioned.
Reaction temperature: 100 to 350°C,
Pressure: 0 to 0.5 MPaG
Molar ratio of hydrogen to nitrobenzene: 10 to 30:1
Gas superficial velocity (GHSV: raw material gas flow rate per unit superficial volume): 1,000 to 2,000 h⁻¹

By producing aniline under such reaction conditions, the catalyst of the present invention shows a raw material conversion of at least 99%, further nearly 100%, and shows an aniline selectivity of at least 99%, further at least 99.5%, still further at least 99.7%.

Further, the catalyst of the present invention can be used in a method for producing an aromatic amine compound other than the method for producing aniline by hydrogenating nitrobenzene, or in a method for hydrogenating an aromatic nitro compound other than nitrobenzene. For example, the catalyst of the present invention can be used in methods of hydrogenating alkyl-substituted nitrobenzenes, nitrophenols, nitropyridines, nitrochlorobenzenes, etc., and in methods for producing aromatic amine compounds by hydrogenating them.

### EXAMPLES

Now, the present invention will be described in detail with reference to Examples. However, the present invention is not limited to these Examples.

### (BET specific surface area)

The BET specific surface area of a sample was measured by a BET 1-point method. For the measurement, a usual BET measuring device (device name: II2300, Flow Soap manufactured by Shimadzu Corporation) was used.

Prior to the measurement of the BET specific surface area, the sample was pretreated by treatment at 150°C for 30 minutes in a nitrogen gas stream.

### (Composition analysis)

The composition analysis of the sample was carried out by an ICP method. For the measurement, a common ICP device (device name: Optima5300DV, manufactured by Perkin Elmer) was used.

Prior to the measurement, the sample was dissolved and pretreated with a hydrofluoric acid aqueous solution, and the obtained sample aqueous solution was measured by an ICP method.

### (Coking rate)

The coking rate of a sample was obtained by the following formula from the change in catalyst weight as between before and after the hydrogenation reaction. Coking rate (g/hr/L) = Weight increase of the composition (g)/reaction time (hr)/catalyst volume (L)

### (Differential pressure of catalyst layer)

The differential pressure of the catalyst layer was obtained from the following formula. Differential pressure of the catalyst layer=(pressure at the catalyst layer inlet) - (pressure at the catalyst layer outlet)

As to the pressures at the catalyst layer inlet and outlet, the pressures at the time when nitrogen gas was flown at 5 NL/min at a catalyst layer temperature of 175°C, were measured by a water column manometer.

### EXAMPLE 1

### (Preparation of catalyst)

578 mL of ion-exchanged water, 43.7 g of amorphous silica powder and 13.5 g of slaked lime powder were charged into a 2 L glass container, and mixed and stirred at 40°C, to prepare a calcium silicate slurry.

On the other hand, 341.5 g of a supported metal aqueous solution comprising 10.2 wt% of Cu and 2.5 wt% of Zn, was prepared by using nitrates of the respective metals, as water-soluble metal salts.

Then, while stirring the calcium silicate slurry, the supported metal aqueous solution was added at 40°C to obtain a mixed slurry. At the time of addition of the supported metal aqueous solution, using a 20% aqueous sodium carbonate solution, the pH of the calcium silicate slurry was adjusted to 7.0±0.5. Here, the addition time of the supported metal aqueous solution was 6 hours.

The mixed slurry was subjected to solid-liquid separation, and the obtained cake was reslurry-washed with ion-exchanged water. The cake after washing was dried in air at 110°C overnight, to obtain a dried powder. The dried powder and 2.4 g of graphite were mixed. Then, the obtained mixed powder was molded into a disk-shaped molded product of 20 mm in diameter and 5 mm in length by using a press molding machine. The density of the obtained molded product was 1.23 g/mL. The molded product was calcined at 700°C for 3 hours, to obtain a composition in this Example.

The BET specific surface area of the composition was 9.8 m²/g.

### (Hydrogenation reaction of nitrobenzene)

Using the composition in this Example as a catalyst, a hydrogenation reaction of nitrobenzene was carried out.

The composition in this Example was pulverized and sieved to obtain particles of indefinite shape having a particle size of from 1.0 to 2.8 mm, which were used as catalyst particles. The catalyst particles were filled in a fixed bed reactor (diameter: 21 mm, catalyst layer volume: 30 mL). Then, the catalyst particles were activated by subjecting the catalyst particles to reduction treatment under a hydrogen gas flow at from 180 to 215°C. Then, a hydrogenation reaction of nitrobenzene was carried out by flowing through the catalyst particles a mixed gas of hydrogen and nitrobenzene preheated to 175°C, wherein the molar ratio of hydrogen to nitrobenzene was 15:1. In the hydrogenation reaction, GHSV was 1,500 h⁻¹, and the reaction pressure was 0.14 MPaG.

Upon expiration of 2,800 hours, the nitrobenzene conversion was 100%, and the aniline selectivity was 99.86%.

The differential pressure of the catalyst layer before the reaction (at the initiation of the reaction) was 10 mmH₂O. Whereas, even after the reaction of 2,800 hours, there was no change in the differential pressure of the catalyst layer. Further, after the completion of the reaction, the catalyst was visually observed. As a result, no fixing of catalyst particles to one another by coking was observed. Here, the coking rate was 2.5×10⁻² g/hr/L.

### EXAMPLE 2

The composition in this Example was obtained in the same manner as in Example 1 except that a supported metal aqueous solution comprising 9.7 wt% of Cu, 1.2 wt% of Zn and 0.8 wt% of Al, was used in an amount of 398.5 g, and a hydrogenation reaction of nitrobenzene using the composition was carried out. Here, the density of the molded product before calcining was 1.20 g/mL.

Upon expiration of 2,500 hours, the nitrobenzene conversion was 100%, and the aniline selectivity was 99.85%. Further, while the differential pressure of the catalyst layer before the reaction was 9 mmH₂O, the differential pressure of the catalyst layer after the reaction was 10 mmH₂O, i.e. the increase in differential pressure was slight.

After the completion of the reaction, no fixing of catalyst particles to one another by coking was observed. Here, the coking rate was 2.0×10⁻² g/hr/L.

### EXAMPLE 3

The composition in this Example was obtained in the same manner as in Example 1 except that a supported metal aqueous solution comprising 11.8 wt% of Cu and 1.2 wt% of Zn, was used in an amount of 408.5 g and the calcining temperature was changed to 750°C, and a hydrogenation reaction of nitrobenzene using the composition was carried out. Here, the density of the molded product before calcining was 1.35 g/mL.

Upon expiration of 4,500 hours, the nitrobenzene conversion was 100%, and the aniline selectivity was good at a level of 99.90%.

The differential pressure of the catalyst layer was 8 mmH₂O before the initiation of the reaction, while no change was observed even after the reaction for 4,500 hours.

### EXAMPLE 4

The composition in this Example was obtained in the same manner as in Example 1 except that a supported metal aqueous solution comprising 11.1 wt% of Cu, 1.1 wt% of Zn and 0.5 wt% of Mg, was used in an amount of 456.0 g, and a hydrogenation reaction of nitrobenzene using the composition was carried out. Here, the density of the molded product before calcining was 1.40 g/mL.

Upon expiration of 2,500 hours, the nitrobenzene conversion was 100%, and the aniline selectivity was good at a level of 99.86%. Further, while the differential pressure of the catalyst layer before the reaction was 9 mmH₂O, the differential pressure of the catalyst layer after the reaction was 10 mmH₂O, i.e. the increase in differential pressure was slight.

### EXAMPLE 5

517 mL of ion-exchanged water, 39.1 g of amorphous silica powder and 12.1 g of slaked lime powder were charged into a 2 L glass container, and mixed and stirred, to prepare a calcium silicate slurry.

On the other hand, 153.4 g of a supported metal aqueous solution comprising 11.8 wt% of Cu, 2.0 wt% of Zn and 0.3 wt% of Mg, was prepared by using water-soluble metal salts.

The composition in this Example was obtained in the same manner as in Example 1 except that the obtained calcium silicate slurry and supported metal aqueous solution were used, and a hydrogenation reaction of nitrobenzene using the composition was carried out. The density of the molded product before calcining was 1.20 g/mL.

Upon expiration of 1,800 hours, the nitrobenzene conversion was 100%, and the aniline selectivity was 99.92%. Further, while the differential pressure of the catalyst layer before the reaction was 8 mmH₂O, the differential pressure of the catalyst layer after the reaction was 9 mmH₂O, i.e. the increase in differential pressure was slight.

### EXAMPLE 6

The composition in this Example was obtained in the same manner as in Example 4 except that the calcining temperature was changed to 700°C, and a hydrogenation reaction of nitrobenzene using the composition was carried out. The density of the molded product before calcining was 1.40 g/mL.

Upon expiration of 900 hours, the nitrobenzene conversion was 100%, and the aniline selectivity was 99.71%. Further, while the differential pressure of the catalyst layer before the reaction was 8 mmH₂O, the differential pressure of the catalyst layer after the reaction was 9 mmH₂O, i.e. the increase in differential pressure was slight.

### COMPARATIVE EXAMPLE 1

The composition in this Comparative Example was obtained in the same manner as in Example 1 except that a supported metal aqueous solution comprising 13.2 wt% of Cu, was used in an amount of 328.2 g and the calcining was conducted at 800°C for 3 hours, and a hydrogenation reaction of nitrobenzene using the composition was carried out. The density of the molded product before calcining was 1.45 g/mL.

Upon expiration of 1,300 hours, the nitrobenzene conversion was 100%, and the aniline selectivity was 99.74% i.e. slightly lower than in Examples. Further, the differential pressure of the catalyst layer before the reaction was 8 mmH₂O, but after the reaction, it increased to 15 mmH₂O.

In the catalyst after the completion of the reaction, firm fixation of catalyst particles to one another due to coking was observed. Here, the coking rate was 20.5×10⁻² g/hr/L, i.e. larger than in Examples.

### COMPARATIVE EXAMPLE 2

The composition in this Comparative Example was obtained in the same manner as in Example 1 except that a supported metal aqueous solution comprising 13.2 wt% of Cu, was used in an amount of 81.6 g and the calcining temperature was changed to 500°C, and a hydrogenation reaction of nitrobenzene using the composition was carried out. The density of the molded product before calcining was 0.80 g/mL.

Upon expiration of 800 hours, the nitrobenzene conversion was 100%, and the aniline selectivity was 99.63% i.e. lower than in Examples. Further, the differential pressure of the catalyst layer before the reaction was 8 mmH₂O, but after the reaction, it increased to 13 mmH₂O.

In the catalyst after the completion of the reaction, fixing of catalyst particles to one another due to coking was observed. Here, the coking rate was 23.3×10⁻² g/hr/L, i.e. larger than in Examples.

Table 1 shows the evaluation results of the compositions in Examples and Comparative Examples, and Table 2 shows the results of hydrogenation reaction tests.

### COMPARATIVE EXAMPLE 3

The composition in this Comparative Example was obtained in the same manner as in Example 4 except that the calcining temperature was changed to 650°C, and a hydrogenation reaction of nitrobenzene using the composition was carried out. The density of the molded product before calcining was 1.40 g/mL.

Upon expiration of 650 hours, the nitrobenzene conversion was 100%, and the aniline selectivity was 99.75%. Further, the differential pressure of the catalyst layer before the reaction was 8 mmH₂O, but after the reaction, it increased to 18 mmH₂O.

In the catalyst after the completion of the reaction, fixing of catalyst particles to one another due to coking was observed. Here, the coking rate was 19.3×10⁻² g/hr/L, i.e. larger than in Examples.

**[Table 1]**

| | Cu (wt%) | Zn (wt%) | Ca (wt%) | Mg (wt%) | Al (wt%) | BET specific surface area (m²/g) | Density (g/mL) |
|---|---|---|---|---|---|---|---|
| Example 1 | 31.1 | 8.7 | 6.6 | 0 | 0 | 9.8 | 2.10 |
| Example 2 | 31.6 | 5.5 | 6.5 | 0 | 3.3 | 11.0 | 2.10 |
| Example 3 | 40.5 | 4.5 | 6.1 | 0 | 0 | 7.5 | 1.95 |
| Example 4 | 39.5 | 4.3 | 6.0 | 0.4 | 0 | 8.5 | 1.89 |
| Example 5 | 23.8 | 4.0 | 8.4 | 0.4 | 0 | 6.8 | 1.80 |
| Example 6 | 39.5 | 4.3 | 6.0 | 0.4 | 0 | 23 | 1.80 |
| Comparative Example 1 | 39.9 | 0 | 6.2 | 0 | 0 | 5.4 | 1.80 |
| Comparative Example 2 | 16.0 | 0 | 9.5 | 0 | 0 | 242 | 0.80 |
| Comparative Example 3 | 39.5 | 4.3 | 6.0 | 0.4 | 0 | 51 | 1.45 |

**[Table 2]**

| | Reaction time (hr) | Nitrobenzene conversion rate (%) | Aniline selectivity (%) | Differential pressure of catalyst layer | Coking rate (×10⁻² g/hr/L) |
|---|---|---|---|---|---|
| | | | | At initiation of reaction→after reaction (mmH₂O) | |
| Example 1 | 2800 | 100 | 99.86 | 10→10 | 2.5 |
| Example 2 | 2500 | 100 | 99.85 | 9→10 | 2.0 |
| Example 3 | 4500 | 100 | 99.90 | 8→8 | 2.0 |
| Example 4 | 2500 | 100 | 99.86 | 9→10 | 2.0 |
| Example 5 | 1800 | 100 | 99.92 | 8→9 | 4.5 |
| Example 6 | 900 | 100 | 99.71 | 8→9 | 8.1 |
| Comparative Example 1 | 1300 | 100 | 99.74 | 8→15 | 20.5 |
| Comparative Example 2 | 800 | 100 | 99.63 | 8→13 | 23.3 |
| Comparative Example 3 | 650 | 100 | 99.75 | 8→18 | 19.3 |

As shown in above Tables 1 and 2, the compositions of the present invention whereby the raw material conversion was 100%, were confirmed to have catalytic properties equal to the conventional catalysts. Further, with the compositions of the present invention, the increase in differential pressure of the catalyst layer from the initiation of the hydrogenation reaction of nitrobenzene till expiration of 900 hours or more was at most 1 mmH₂O. Further, the coking rate in each composition was at most 0.1 g/hr/L, and thus, it was confirmed that coking was remarkably less. In contrast, in the catalysts in Comparative Examples, even in the reaction of at most 800 hours, the differential pressure of the catalyst layer was at least 5 mmH₂O, and yet, the coking rate was at least 19 g/hr/L, i.e. coking resistance characteristics were low. From these results, it was confirmed that with the composition of the present invention, it is possible to conduct the hydrogenation reaction over a long period of time without causing an increase in the differential pressure.

### EXAMPLE 7

A hydrogenation reaction of nitrobenzene was carried out by using the catalyst of the present invention and a catalyst comprising copper and calcium silicate.

That is, a calcium silicate slurry was prepared by mixing and stirring 578 mL of ion-exchanged water, 43.7 g of amorphous silica powder and 13.5 g of slaked lime powder. On the other hand, 328.2 g of a supported metal aqueous solution containing 13.2 wt% of Cu was prepared.

Then, while stirring the calcium silicate slurry, the supported metal aqueous solution was added at 40°C to obtain a mixed slurry. At the time of addition of the supported metal aqueous solution, using a 20% aqueous sodium carbonate solution, the pH of the calcium silicate slurry was adjusted to 7.0±0.5. Here, the addition time of the supported metal aqueous solution was 6 hours.

The obtained mixed slurry was subjected to solid-liquid separation, and the obtained cake was reslurry-washed with ion-exchanged water. The cake after washing was dried at 110°C overnight in air, to obtain a dried powder. The obtained dried powder was mixed with 2.4 g of graphite, followed by press molding to obtain a disk-shaped molded product having a diameter of 20 mm × a length of 5 mm. The molded product was calcined at 500°C for 3 hours to obtain a catalyst comprising copper and calcium silicate. The BET specific surface area of the obtained catalyst was 196 m²/g.

Using a fixed bed reactor similar to the reactor used in Example 1, a catalyst prepared in the same manner as in Example 4 was filled to 17 mL of the upper portion of the reactor, which was used as an upper catalyst. On the other hand, the obtained catalyst comprising copper and calcium silicate was filled at 13 mL of the lower portion of the reactor, which was used as a middle-lower catalyst. Thus, a catalyst layer comprising the upper catalyst and the middle lower catalyst was formed.

Except for using the catalyst layer, a hydrogenation reaction of nitrobenzene was carried out in the same manner as in Example 1.

Upon expiration of 4,200 hours, the nitrobenzene conversion was 100%, and the aniline selectivity was 99.81%. Further, the differential pressure of the catalyst layer before the reaction was 9 mmH₂O, and no change was observed even after the reaction. In the catalyst after the reaction, no fixing of particles to one another due to coking was observed, and the coking rate was 2.0×10⁻² g/hr/L in the catalyst at the upper portion prepared in Example 4, and was 1.4×10⁻² g/hr/L in the catalyst at the lower portion prepared in this Example.

### INDUSTRIAL APPLICABILITY

The composition of the present invention can be used as a catalyst, specifically as a hydrogenation catalyst, more specifically as a hydrogenation catalyst for an aromatic nitro compound, for example, as a catalyst at the time of producing aniline by hydrogenating an aromatic nitro compound, and it is particularly useful as a catalyst in the field of hydrogenation reactions of aromatic nitro compounds.

## Claims

1. A hydrogenation catalyst consisting essentially of:
from 20 to 50 wt% of copper,
from 1 to 15 wt% of zinc,
optionally an auxiliary catalyst component selected from the group consisting of magnesium, strontium, barium and aluminum, and
calcium silicate wherein the molar ratio of calcium oxide to silicon oxide in the calcium silicate is from 0.1 to 0.7,
said hydrogenation catalyst having a BET specific surface area of from 3 to 30 m²/g.

2. The hydrogenation catalyst according to Claim 1, which contains at least one member selected from the group consisting of magnesium, strontium, barium and aluminum.

3. A method for producing the hydrogenation catalyst as defined in Claim 1 or 2, **characterized by** comprising
a mixing step of contacting calcium silicate with an aqueous solution containing copper and zinc, to obtain a mixture,
a molding step of molding the mixture, and
a calcination step of calcining at a temperature of at least 680°C and less than 800°C for 1 hour to 24 hours.

4. A method for producing an aromatic amine compound, which comprises contacting the hydrogenation catalyst as defined in Claim 1 or 2 with raw material comprising an aromatic nitro compound and hydrogen.

## Patentansprüche

1. Hydrierungskatalysator, der im Wesentlichen besteht aus:
zwischen 20 und 50 Gew.-% Kupfer,
zwischen 1 und 15 Gew.-% Zink,
wahlweise einer Hilfskatalysatorkomponente, die ausgewählt ist aus der Gruppe, die besteht aus Magnesium, Strontium, Barium und Aluminium, und
Calciumsilicat, wobei das Molverhältnis von Calciumoxid zu Siliziumoxid in dem Calciumsilicat zwischen 0,1 und 0,7 beträgt,
der Hydrierungskatalysator eine BET-spezifische Oberfläche von zwischen 3 und 30 m²/g aufweist.

2. Hydrierungskatalysator nach Anspruch 1, der mindestens ein Element enthält, das ausgewählt ist aus der Gruppe, die besteht aus Magnesium, Strontium, Barium und Aluminium.

3. Verfahren zur Herstellung des Hydrierungskatalysators nach Anspruch 1 oder 2, **dadurch gekennzeichnet, dass** es umfasst:
einen Schritt des Mischens zum Inkontaktbringen von Calciumsilikat mit einer wässrigen Lösung, die Kupfer und Zink enthält, um ein Gemisch zu erhalten,
einen Schritt des Formens zum Formen des Gemischs, und
einen Schritt des Kalzinierens zum Kalzinieren bei einer Temperatur von mindestens 680 °C und niedriger als 800 °C während 1 Stunde bis 24 Stunden.

4. Verfahren zur Herstellung einer aromatischen Aminverbindung, welches das Inkontaktbringen des Hydrierungskatalysators nach Anspruch 1 oder 2 mit Rohstoff umfasst, der eine aromatische Nitroverbindung und Wasserstoff umfasst.

## Revendications

1. Catalyseur d'hydrogénation consistant essentiellement en :
20 à 50 % en poids de cuivre,
1 à 15 % en poids de zinc,
éventuellement un composant de catalyseur auxiliaire choisi dans le groupe constitué par le magnésium, le strontium, le baryum et l'aluminium, et
du silicate de calcium, dans lequel le rapport molaire de l'oxyde de calcium à l'oxyde de silicium dans le silicate de calcium est de 0,1 à 0,7,
ledit catalyseur d'hydrogénation ayant une surface spécifique BET de 3 à 30 m²/g.

2. Catalyseur d'hydrogénation selon la revendication 1, qui contient au moins un membre choisi dans le groupe constitué par le magnésium, le strontium, le baryum et l'aluminium.

3. Procédé pour produire le catalyseur d'hydrogénation tel que défini dans la revendication 1 ou 2, **caractérisé en ce qu'**il comprend
une étape de mélange dans laquelle du silicate de calcium est mis en contact avec une solution aqueuse contenant du cuivre ou du zinc, pour que soit obtenu un mélange,
une étape de moulage dans laquelle le mélange est moulé, et
une étape de calcination dans laquelle une calcination à une température d'au moins 680°C et inférieure à 800°C est effectuée pendant 1 heure à 24 heures.

4. Procédé pour produire un composé amine aromatique, qui comprend la mise en contact du catalyseur d'hydrogénation tel que défini dans la revendication 1 ou 2 avec une matière première comprenant un composé nitro aromatique et de l'hydrogène.
